# FASCICULE DE BREVET EUROPEEN

(11) **EP 0 738 503 B1**
(45) Date de publication et mention de la délivrance du brevet: **05.12.2001**
(21) Numéro de dépôt: 96420128.9
(22) Date de dépôt: 16.04.1996
(51) Int. Cl.: A61F 2/30, A61F 2/36

(54) **Prothèse de hanche à joint d'étanchéité**
Hüftprothese mit abgedichtetem Gelenk
Hip prosthesis having a sealed joint

(30) Priorité: 18.04.1995 FR 9504773
(43) Date de publication de la demande: 23.10.1996
(73) Titulaire: Wright Cremascoli Orthotechnique, 94000 Créteil (FR); Brunet, Jean-Claude, 75017 Paris (FR); Chapuis, Patrick, 43000 Espaly Saint Marcel (FR); De Christen, Loic, 92310 Sèvres (FR); Laine, Marc, 03400 Yzeure (FR); Lelong, Pierre, 92200 Neuilly sur Seine (FR); Massin, Philippe, 49100 Angers (FR); Michaut, Emmanuel, 76400 Fécamp (FR); MISSENARD, Gilles, 75016 Paris (FR); Pages, Christian, 61100 Flers de l'Orne (FR); Richter, Dominique, 1400O Caen (FR); Serrault, Michel, 61100 Flers de L'orne (FR); Van Nedervelde, Thierry, 14500 Vire (FR)
(72) Inventeur: Brunet, Jean-Claude, 75017 Paris (FR); Chapuis, Patrick, 43000 Espaly Saint Marcel (FR); De Christen, Loic, 92310 Sèvres (FR); Laine, Marc, 03400 Yzeure (FR); Lelong, Pierre, 92200 Neuilly sur Seine (FR); Massin, Philippe, 49100 Angers (FR); Michaut, Emmanuel, 76400 Fécamp (FR); Missenard, Gilles, 75016 Paris (FR); Pages, Christian, 61100 Flers de l'Orne (FR); Richter, Dominique, 14000 Caen (FR); Serrault, Michel, 61100 Flers de l'Orne (FR); Van Nedervelde, Thierry, 14500 Vire (FR); Tanguy, Christian, 91860 Epinay sous Sénart (FR); Lavaste, François, 91240 Saint Michel sur Orge (FR)
(74) Mandataire: Poncet, Jean-François

(56) Documents cités:
- EP-A- 0 323 800
- WO-A-95/10990
- FR-A- 2 668 058
- US-A- 4 199 864
- US-A- 4 636 218

## Description

La présente invention concerne les prothèses de hanche destinées à former la partie mâle de l'articulation entre le fémur humain et le bassin.

On connaît déjà un grand nombre de prothèses de hanche destinées à être implantées en extrémité supérieure du fémur, et comprenant une tête de prothèse reliée par un col à une tige de prothèse conformée pour se loger dans un canal longitudinal aménagé dans le fémur. La tête et le col constituent une zone extra osseuse destinée à rester hors de l'os et se raccordant par une zone de transition à la tige constituant une zone intra-osseuse de fixation.

La fixation de la tige dans l'os est actuellement réalisée par deux techniques distinctes : selon la technique la plus ancienne, la tige est collée dans le canal du fémur par un ciment occupant l'espace entre la tige et le canal. Dans ce cas, la tige présente éventuellement des nervures ou rainures d'ancrage dans le ciment. Selon la deuxième technique, plus récente, telle que décrite par exemple dans le document FR-A-2 668 058, on pose la prothèse sans ciment, en utilisant la capacité de certains matériaux à permettre la formation d'os à l'interface entre la prothèse et le corps humain. On prévoit alors, sur la partie intra-osseuse de tige, au moins une zone de fixation dont la surface extérieure est en matériau poreux réhabitable. La formation d'os assure alors le scellement de la prothèse, pour sa fixation.

Un problème de toutes les prothèses de hanche connues, quel que soit le procédé de fixation, réside dans la longévité insuffisante des prothèses. On constate en effet, à l'issue d'une durée qui peut être variable en fonction des sujets et en fonction des techniques utilisées, une dégradation du scellement de la prothèse ou de l'os périprothétique, notamment consécutivement à un processus d'ostéolyse.

Le problème proposé par la présente invention est de réduire ou de supprimer totalement ces phénomènes d'ostéolyse se produisant au bout d'un certain temps autour des tiges de prothèse de hanche.

L'idée qui est à la base de l'invention est que, dans une zone d'articulation, l'usure progressive des matériaux formant l'articulation artificielle produit des micro particules de corps étrangers susceptibles de pénétrer dans la zone de fixation entre la tige de prothèse et le ciment ou entre le ciment et l'os, lorsqu'existe un certain jeu en zone périphérique de la prothèse entre la tige, le ciment et l'os, notamment lors de contraintes mécaniques appliquées sur le membre muni de la prothèse. Lors des contraintes mécaniques, des déformations différentielles peuvent vraisemblablement se produire entre la tige de prothèse et l'os, créant momentanément des espaces dans lesquels peuvent pénétrer les micro particules étrangères. Ces micro particules étrangères provoqueraient ensuite une réaction de l'organisme, sous forme d'une ostéolyse, qui réduirait progressivement la solidité de l'ancrage de prothèse dans l'os.

Selon un aspect essentiel, l'invention se propose de créer une barrière naturelle empêchant la pénétration des micro particules étrangères dans la zone de fixation de prothèse. Selon l'invention, cet effet doit être obtenu de la façon la plus naturelle possible, sans augmenter les structures étrangères au corps humain.

En complément, l'invention se propose de réduire les risques de déformation différentielle entre la tige de prothèse et l'os, évitant ainsi l'apparition d'interstices à l'interface entre la prothèse et l'os, interstices susceptibles d'accueillir des micro-particules de corps étrangers.

Pour atteindre ces objets ainsi que d'autres, selon le premier aspect de l'invention, la prothèse de hanche comprend une tête de prothèse reliée par un col à une tige de prothèse conformée pour se loger et être collée par un ciment dans un canal longitudinal aménagé dans le fémur, la tête et le col constituant une zone extra osseuse destinée à rester hors de l'os et se raccordant, par une zone de transition, à la tige constituant une zone intra-osseuse ; au voisinage de la zone de transition, la zone extra osseuse comprend une surface annulaire constituée d'une matière présentant une affinité avec l'os ou avec les membranes fibreuses néoformées qui se forment naturellement autour des corps étrangers dans le corps humain, de sorte que, après la pose de la prothèse, il se forme naturellement un joint d'étanchéité fibreux entre l'extrémité supérieure du fémur et ladite surface annulaire, le joint d'étanchéité fibreux ainsi formé s'opposant à la pénétration de corps étrangers et produits d'usure entre l'os et la tige de prothèse.

Selon un mode de réalisation avantageux, la surface annulaire de zone extra osseuse se prolonge, au-delà de la zone de transition, par une surface annulaire de zone intra-osseuse également en une matière présentant une affinité avec l'os ou avec les membranes fibreuses néoformées qui se forment naturellement autour des corps étrangers, permettant la réimplantation et le tassement d'os spongieux entre la prothèse et l'os cortical du fémur, constituant un joint osseux en complément et en continuité du joint d'étanchéité fibreux et avant la zone de fixation cimentée de tige de prothèse.

Selon le second aspect de l'invention, permettant de réduire les déformations différentielles entre la tige de prothèse et l'os, la zone intra-osseuse de prothèse comprend une partie proximale métaphysaire anatomique à section transversale en triangle curviligne à flancs évidés, définissant trois zones de contact privilégiées distinctes et écartées les unes des autres, à savoir une première zone de contact sur la corticale interne de l'os, une seconde zone de contact sur la corticale postéro externe de l'os, et une troisième zone de contact sur la corticale antéro externe de l'os, les seconde et troisième zones de contact étant reliées à la première zone de contact par des flancs concaves évidés destinés à rester à l'écart de l'os, les trois zones de contact s'inscrivant dans le contour anatomique interne de l'os cortical.

Selon un mode de réalisation préféré, la zone intra-osseuse de prothèse comprend une partie distale en forme générale de cône de révolution à axe rectiligne, de préférence à surface périphérique lisse et polie.

On notera que les caractéristiques de l'invention permettant la constitution d'un joint d'étanchéité peuvent être utilisées indépendamment des caractéristiques relatives à la forme géométrique, et réciproquement. En d'autres termes, une prothèse de hanche munie des moyens produisant la formation d'un joint d'étanchéité présentera une longévité nettement améliorée, même lorsqu'elle est dépourvue des caractéristiques particulières de forme géométrique de partie proximale métaphysaire à section transversale en triangle curviligne. Cependant, la combinaison de ces moyens de joint d'étanchéité avec une section transversale en triangle curviligne améliorera encore la longévité, car la forme géométrique particulière permet de réduire les mouvements différentiels entre la prothèse et l'os, et cette réduction de mouvements favorise l'apparition des membranes fibreuses néoformées formant le joint d'étanchéité entre la prothèse et l'os, et réduit les risques de pénétration de particules étrangères entre la prothèse et l'os.

Réciproquement, une prothèse pourra être munie des caractéristiques géométriques de forme de la partie métaphysaire à section transversale en triangle curviligne, donnant à la prothèse une meilleure tenue mécanique, et une meilleure longévité grâce à la réduction des mouvements différentiels et des risques de pénétration de particules étrangères entre la tige de prothèse et l'os, même lorsque cette prothèse est dépourvue des moyens provoquant l'apparition d'un joint d'étanchéité.

D'autres objets, caractéristiques et avantages de la présente invention ressortiront de la description suivante de modes de réalisation particuliers, faite en relation avec les figures jointes, parmi lesquelles:
- la figure 1 est une vue de face d'une prothèse selon un mode de réalisation de la présente invention ;
- la figure 2 illustre la première étape de pose d'une prothèse de la figure 1 ;
- la figure 3 illustre la seconde étape de pose de la prothèse de la figure 1 ;
- la figure 4 illustre la prothèse de la figure 1, après pose et formation du joint d'étanchéité ;
- la figure 5 illustre, en vue de face, les caractéristiques de forme de la partie métaphysaire d'une prothèse de hanche selon un mode de réalisation de l'invention ;
- la figure 6 illustre en vue de côté la prothèse de la figure 5, et montre simultanément les formes de coupe transversale de la prothèse en différentes zones ; et
- la figure 7 illustre, en coupe transversale, la répartition des zones d'appui de la prothèse dans l'os.

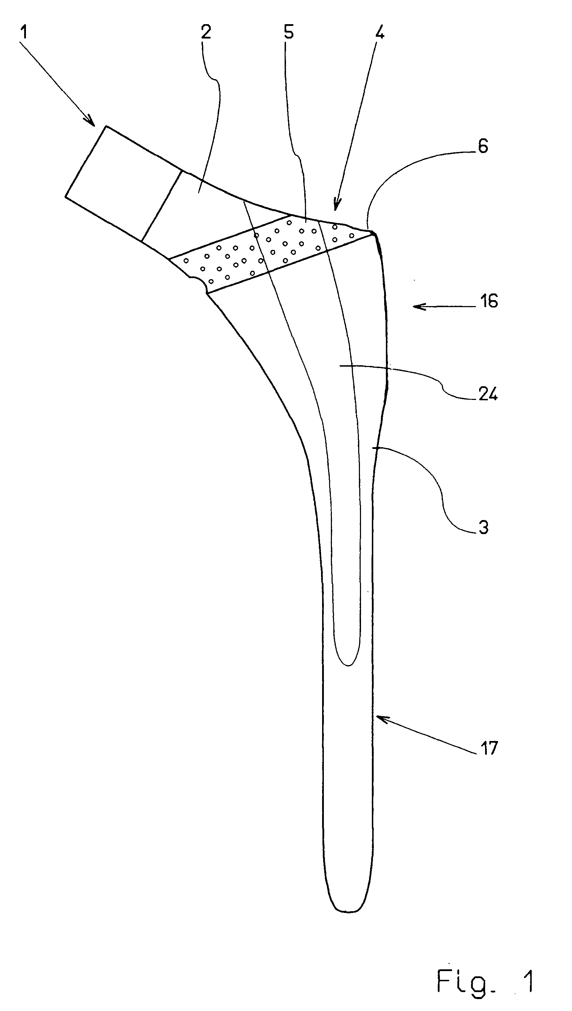

Dans le mode réalisation illustré sur la figure 1, la prothèse de hanche selon l'invention, destinée à former la partie mâle de l'articulation du fémur, est destinée à être implantée en extrémité supérieure du fémur. Elle comprend une tête de prothèse 1 reliée par un col 2 à une tige de prothèse 3 conformée pour se loger et être collée par un ciment dans un canal longitudinal aménagé dans le fémur.

La tête 1 et le col 2 constituent une zone extra osseuse, destinée à rester hors de l'os, et se raccordant par une zone de transition 4 à la tige 3 constituant une zone intra-osseuse.

Au voisinage de la zone de transition 4, la zone extra osseuse comprend une surface annulaire 5 constituée d'une matière présentant une affinité avec l'os ou avec les membranes fibreuses néoformées qui se forment naturellement autour des corps étrangers dans le corps humain.

Dans le mode de réalisation représenté, la surface annulaire extra osseuse 5 se prolonge, au-delà de la zone de transition 4, par une surface annulaire de zone intra-osseuse 6, également en une matière présentant une affinité avec l'os ou avec les membranes fibreuses néoformées qui se forment naturellement autour des corps étrangers.

Les matières formant les surfaces annulaires 5 et 6 peuvent être des matières identiques ou différentes, telles que des matières poreuses réhabitables, des matières phosphocalciques, des céramiques inertes ou bioactives, etc. On peut avantageusement utiliser une matière poreuse ou rugueuse réhabitable, éventuellement recouverte partiellement d'une substance phosphocalcique.

De préférence, la surface annulaire de zone intra-osseuse 6 est conformée en gorge annulaire, pour favoriser l'insertion d'os spongieux lors de la pose de la prothèse. L'os spongieux est introduit et impacté dans l'espace demeurant vacant entre la surface annulaire de zone intra-osseuse 6 et la paroi corticale périphérique du fémur proximal.

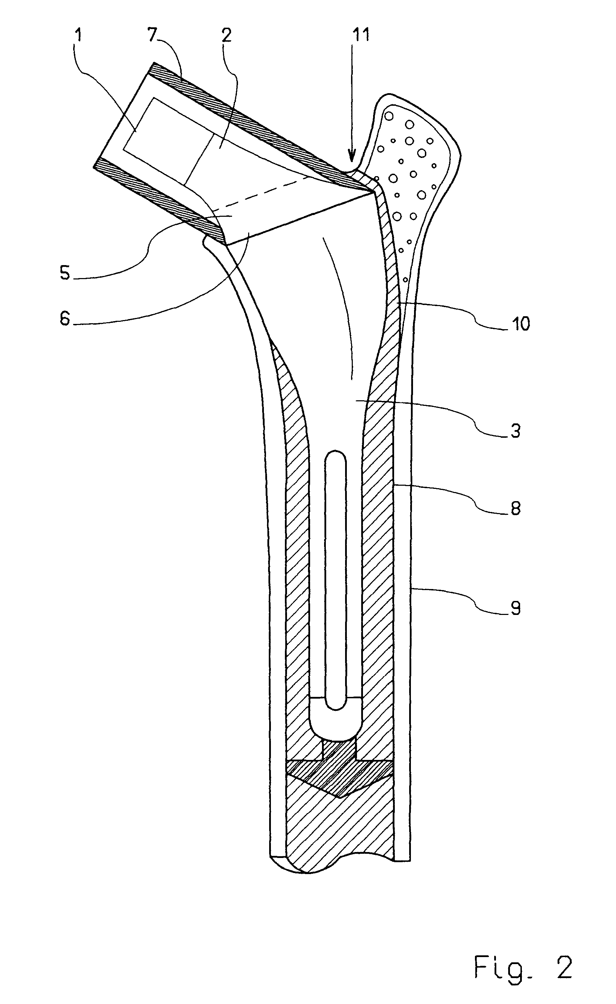

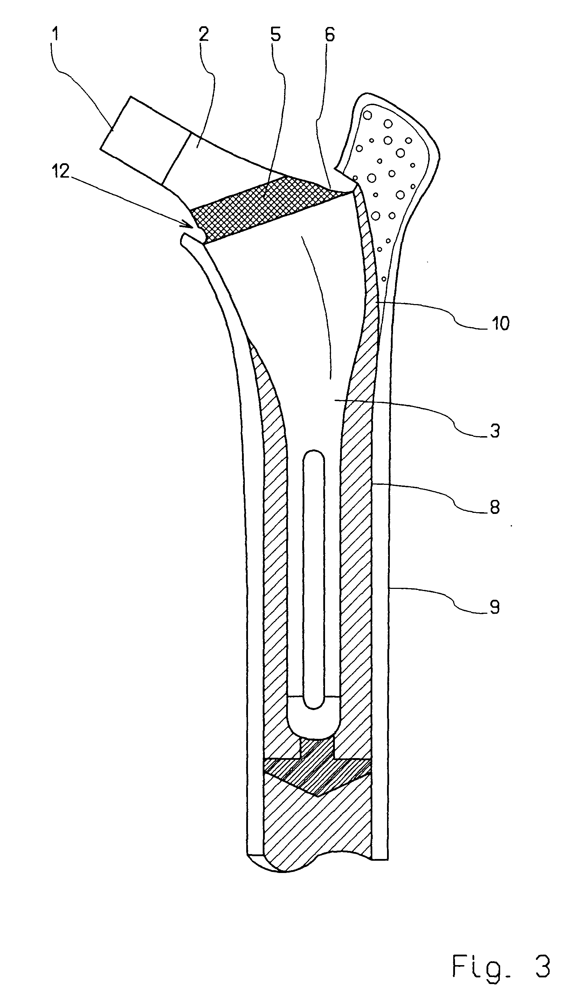

Avant la pose, comme illustré sur la figure 2, un capuchon de protection amovible 7 recouvre la prothèse dans la zone extra osseuse formée par la tête 1 et le col 2, et recouvre la prothèse dans les zones formées par les surfaces annulaires 5 et 6, jusqu'à la zone de fixation à cimenter de la tige 3. Le canal 8 est préalablement aménagé dans l'os 9, de façon connue, en utilisant des outils appropriés. Le ciment 10 est ensuite introduit dans le canal 8, puis la tige 3 est engagée dans le canal 8 pourvu du ciment 10. L'excès de ciment s'échappe par l'ouverture d'extrémité 11, et le capuchon protecteur 7 protège alors les surfaces annulaires 5 et 6 pour éviter leur dégradation par le ciment 10. Après fixation de la prothèse par le ciment, et enlèvement de l'excès de ciment sortant de l'ouverture d'extrémité 11, on peut enlever le capuchon 7 comme illustré sur la figure 3, laissant apparaître les surfaces annulaires 5 et 6 au-delà de la zone de fixation constituée par la partie de tige 3 entourée du ciment 10.

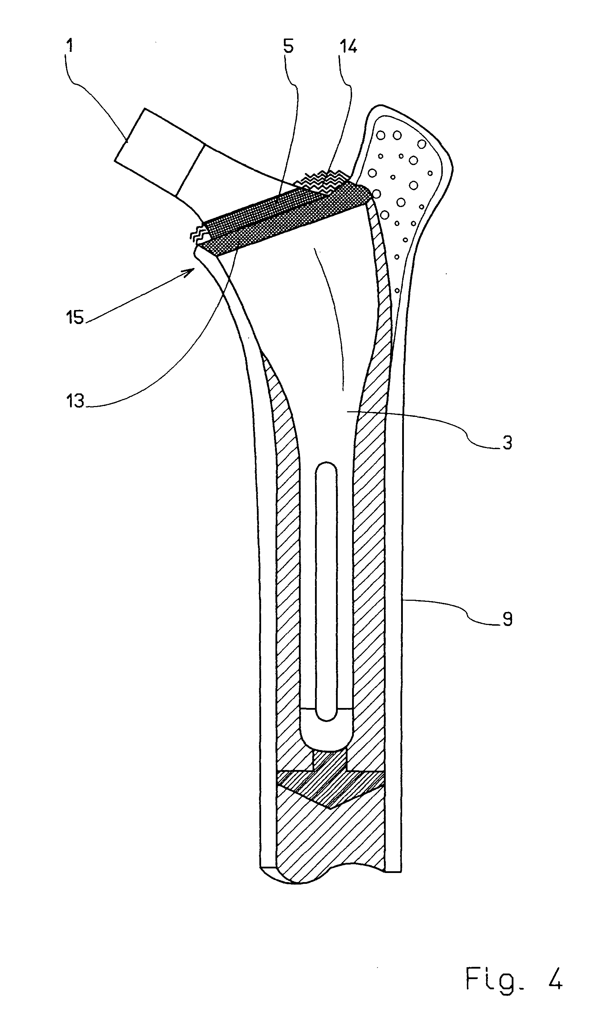

On s'arrange pour ménager, autour de la surface annulaire de zone intra-osseuse 6, un interstice 12. On peut par exemple mettre à profit une forme de surface annulaire de zone intra-osseuse 6 en forme de gorge annulaire. Dans cet interstice 12, on bourre de l'os spongieux qui, par réimplantation d'os spongieux dans la surface annulaire de zone intra-osseuse 6, réalise ensuite un joint d'étanchéité osseux 13, comme illustré sur la figure 4. Simultanément, après la pose de la prothèse, il se forme naturellement un joint d'étanchéité fibreux 14 entre l'extrémité supérieure 15 de l'os 1 et la surface annulaire 5 de zone extra osseuse, le joint d'étanchéité 14 s'opposant à la pénétration de corps étrangers entre l'os 1 et la tige 3 de prothèse.

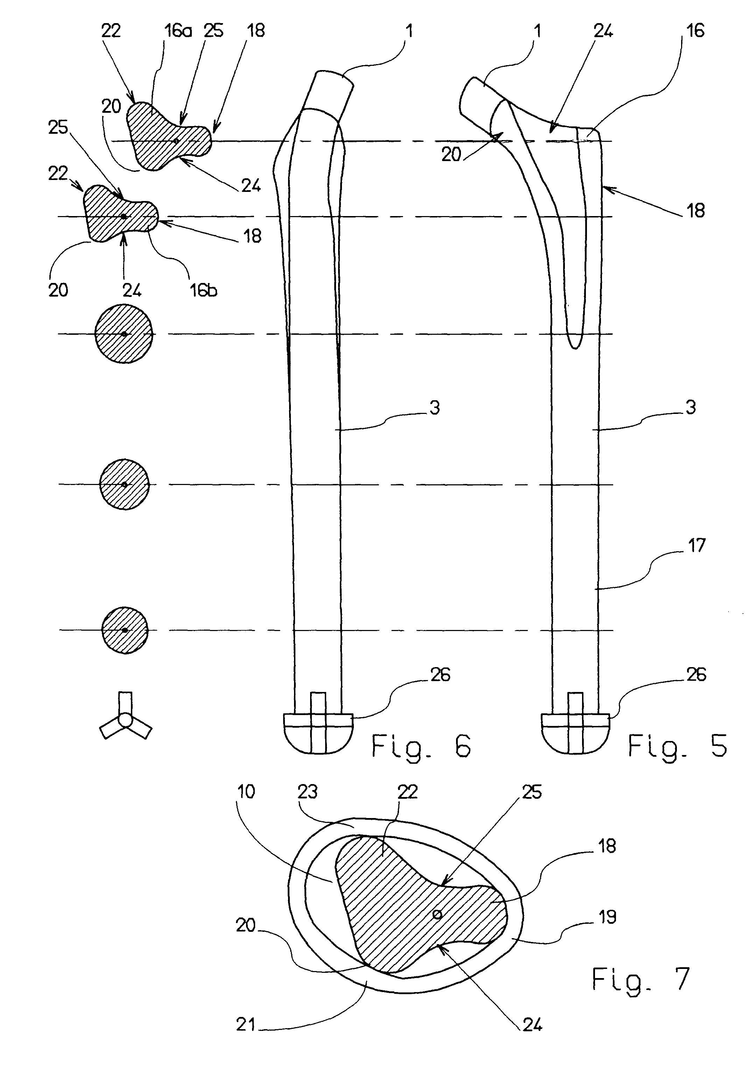

La prothèse selon l'invention peut avantageusement avoir une forme géométrique telle que représentée sur les figures 5 à 7.

Comme illustré sur les figures, la zone intra-osseuse de prothèse comprend une partie proximale métaphysaire 16 anatomique, et une partie distale diaphysaire 17.

La partie proximale métaphysaire 16 présente une section transversale en triangle curviligne à flancs évidés, représentée par exemple par les sections 16a et 16b de la figure 6, définissant trois zones de contact privilégiées distinctes et écartées les unes des autres, à savoir une première zone de contact 18 destinée à venir en contact sur la corticale interne 19 de l'os, une seconde zone de contact 20 destinée à venir porter contre la corticale postéro externe 21 de l'os, et une troisième zone de contact 22 destinée à porter sur la corticale antéro externe 23 de l'os, comme illustré sur la figure 7.

Les seconde zone de contact 20 et troisième zone de contact 22 sont reliées à la première zone de contact 18 par des flancs 24 et 25 concaves évidés, destinés à rester à l'écart de l'os, comme illustré sur la figure 7. Les trois zones de contact 18, 20 et 22 s'inscrivent dans le contour anatomique interne de l'os cortical.

Dans le mode de réalisation représenté sur les figures, la partie distale 17 de tige de prothèse présente une forme générale de cône de révolution à axe rectiligne. Sa surface périphérique est lisse et polie.

Par ces formes géométriques, le contact entre la tige de prothèse et l'os s'effectue selon les trois zones privilégiées de contact 18, 20 et 22, qui sont le plus éloignées possible du centre de la tige, permettant de supporter un couple de blocage en rotation maximum. La présence des flancs évidés 24 et 25 permet d'éviter tout contact avec l'os dans les zones intermédiaires, et de s'assurer ainsi que les contacts se font dans les zones de contact 18, 20 et 22. Les zones intermédiaires peuvent être comblées par du ciment.

La partie distale 17 de section, qui est ronde, lisse et polie, favorise un effet de glissement et de coincement axial de la tige dans l'os, de sorte que les sollicitations sur le ciment 10 sont préférentiellement des sollicitations de compression, tandis que les sollicitations de cisaillement sont principalement supportées par les zones de contact 18, 20 et 22 de partie proximale métaphysaire 16 en appui direct sur l'os cortical.

Un centreur 26, en une matière élastiquement déformable, est adapté en bout de la partie distale 17 de l'implant ou de l'ancillaire correspondant, pour centrer ladite partie distale 17 dans le canal médullaire 8 du fémur, sans perturber les contacts réalisés par les zones de contact 18, 20 et 22 de partie proximale 16 de prothèse.

La présente invention n'est pas limitée aux modes de réalisation qui ont été explicitement décrits, mais elle en inclut les diverses variantes et généralisations contenues dans le domaine des revendications ci-après.

## Revendications

1. Prothèse de hanche destinée à être implantée en extrémité supérieure d'un fémur (9), comprenant une tête de prothèse (1) reliée par un col (2) à une tige de prothèse (3) conformée pour se loger et être collée par un ciment dans un canal longitudinal (8) aménagé dans le fémur (9), la tête (1) et le col (2) constituant une zone extra osseuse destinée à rester hors de l'os (9) et se raccordant par une zone de transition (4) à la tige (3) constituant une zone intra-osseuse, **caractérisée en ce que**, au voisinage de la zone de transition (4), la zone extra-osseuse comprend une surface annulaire (5) constituée d'une matière présentant une affinité avec l'os ou avec les membranes fibreuses néoformées qui se forment naturellement autour des corps étrangers dans le corps humain, de sorte que, après la pose de la prothèse, il se forme naturellement un joint d'étanchéité fibreux (14) entre l'extrémité supérieure (15) du fémur (9) et ladite surface annulaire (5), le joint d'étanchéité (14) fibreux ainsi formé s'opposant à la pénétration de corps étrangers et produits d'usure entre l'os (9) et la tige (3) de prothèse.

2. Prothèse selon la revendication 1, **caractérisée en ce que** la surface annulaire de zone extra osseuse (5) se prolonge, au-delà de la zone de transition (4), par une surface annulaire de zone intra-osseuse (6) en une matière présentant une affinité avec l'os ou avec les membranes fibreuses néoformées qui se forment naturellement autour des corps étrangers, permettant la réimplantation et le tassement d'os spongieux entre la prothèse et l'os cortical du fémur, constituant un joint osseux (13) en complément et en continuité du joint d'étanchéité fibreux (14) et avant la zone de fixation cimentée de tige de prothèse (3).

3. Prothèse selon la revendication 2, **caractérisée en ce que** la surface annulaire de zone intra-osseuse (6) est conformée en gorge annulaire pour favoriser l'insertion d'os spongieux lors de la pose de la prothèse.

4. Prothèse selon l'une quelconque des revendications 1 à 3, **caractérisée en ce que** la surface annulaire (5, 6) est en une matière rugueuse ou poreuse réhabitable, éventuellement recouverte partiellement d'une substance phosphocalcique.

5. Prothèse selon l'une quelconque des revendications 1 à 4, **caractérisée en ce qu'**un capuchon de protection amovible (7) recouvre la prothèse dans la zone extra osseuse (1, 2) et jusqu'à la zone de fixation à cimenter de la tige (3), pour protéger du ciment (10) la surface annulaire périphérique (5, 6) de prothèse destinée à la constitution ultérieure du joint d'étanchéité, le capuchon (7) pouvant être enlevé après fixation de la prothèse par le ciment (10).

6. Prothèse selon l'une quelconque des revendications 1 à 5, **caractérisée en ce que** la zone intra-osseuse de prothèse comprend une partie proximale métaphysaire (16) anatomique à section transversale (16a, 16b) en triangle curviligne à flancs évidés (24, 25), définissant trois zones de contact privilégiées distinctes et écartées les unes des autres, à savoir une première zone de contact (18) sur la corticale interne (19) de l'os, une seconde zone de contact (20) sur la corticale postéro externe (21) de l'os, et une troisième zone de contact (22) sur la corticale antéro externe (23) de l'os, les seconde (20) et troisième (22) zones de contact étant reliées à la première zone de contact (18) par des flancs (24, 25) concaves évidés destinés à rester à l'écart de l'os, les trois zones de contact (18, 20, 22) s'inscrivant dans le contour anatomique interne de l'os cortical (9).

7. Prothèse selon la revendication 6, **caractérisée en ce que** la zone intra-osseuse de prothèse comprend une partie distale (17) en forme générale de cône de révolution à axe rectiligne.

8. Prothèse selon la revendication 7, **caractérisée en ce que** la partie distale (17) présente une surface périphérique lisse et polie.

9. Prothèse selon l'une des revendications 6 à 8, **caractérisée en ce qu'**un centreur (26) en une matière élastiquement déformable est adapté en bout de partie distale (17) de l'implant ou de l'ancillaire correspondant, pour centrer ladite partie distale (17) dans le canal médullaire (8) du fémur (9) sans perturber les contacts réalisés par les zones de contact (18, 20, 22) de partie proximale (16) de prothèse.

## Patentansprüche

1. Hüftprothese zur Implantierung an einem oberen Ende eines Oberschenkelknochens (9), mit einem Prothesenkopf (1), der über einen Hals (2) mit einem Schaft der Prothese (3) verbunden ist, der dazu vorgesehen ist, eingebracht und durch einen Zement eingeklebt zu werden in einen Längskanal (8), der in dem Oberschenkelknochen (9) hergestellt ist, wobei der Kopf (1) und der Hals (2) einen außerhalb des Knochens liegenden Bereich bilden, der dazu vorgesehen ist, außerhalb des Knochens (9) zu verbleiben, und der über einen Übergangsbereich (4) mit dem Schaft (3) verbunden ist, welcher einen im Knochen liegenden Bereich bildet, **dadurch gekennzeichnet, daß** in der Nähe des Übergangsbereichs (4) der außerhalb des Knochens liegende Bereich eine ringförmige Oberfläche (5) aufweist, die aus einem Material besteht, welches eine Affinität mit Knochen oder mit den neu gebildeten fibrösen Membranen aufweist, die sich natürlich um Fremdkörper im menschlichen Körper herumbilden, so daß sich nach dem Einsetzen der Prothese eine dichte, fibröse Verbindung (14) zwischen dem oberen Ende (15) des Oberschenkelknochens (9) und der ringförmigen Oberfläche (5) bildet, wobei sich somit die dichte, fibröse Verbindung (14) dem Eindringen von Fremdkörpern und Verschleißprodukten zwischen den Knochen (9) und den Schaft (3) der Prothese widersetzt.

2. Prothese nach Anspruch 1, **dadurch gekennzeichnet, daß** die ringförmige Oberfläche des außerhalb des Knochens liegenden Bereichs (5) verlängert ist, bis zu dem Übergangsbereich (4), durch eine ringförmige Oberfläche des im Knochen liegenden Bereichs (6), aus einem Material, welches eine Affinität mit Knochen oder mit den neu gebildeten fibrösen Membranen aufweist, die sich natürlich um Fremdkörper herumbilden, was die Reimplantation und das Eindrücken schwammartigen Knochens zwischen die Prothese und den kortikalen Knochen des Oberschenkels ermöglicht, wodurch eine zusätzliche knöcherne Verbindung (13) gebildet ist, und zwar in Verlängerung der dichten, fibrösen Verbindung (14) und vor dem zementierten Befestigungsbereich des Schafts der Prothese (3).

3. Prothese nach Anspruch 2, **dadurch gekennzeichnet, daß** die ringförmige Oberfläche des im Knochen liegenden Bereichs (6) als ringförmige Einkehlung ausgebildet ist, um das Einbringen schwammartigen Knochens beim Einsetzen der Prothese zu erleichtern.

4. Prothese nach einem der Ansprüche 1 bis 3, **dadurch gekennzeichnet, daß** die ringförmige Oberfläche (5, 6) aus einem rehabilitierbaren rauhen oder porösen Material ist, das eventuell teilweise mit einer Substanz aus Phosphorcalcit überzogen ist.

5. Prothese nach einem der Ansprüche 1 bis 4, **dadurch gekennzeichnet, daß** eine abnehmbare Schutzhaube (7) die Prothese in dem außerhalb des Knochen liegenden Bereich (1, 2) bedeckt, und zwar bis zu dem einzuzementierenden Befestigungsbereich des Schafts (3), um die ringförmige äußere Oberfläche (5, 6) der Prothese vor dem Zement (10) zu schützen, die für die spätere Bildung der dichten Verbindung vorgesehen ist, wobei die Haube (7) nach der Befestigung der Prothese durch den Zement (10) abgenommen werden kann.

6. Prothese nach einem der Ansprüche 1 bis 5, **dadurch gekennzeichnet, daß** der im Knochen liegende Bereich der Prothese einen proximalen, metaphysären, anatomischen Teil (16) mit einem dreieckförmigen Querschnitt (16a, 16b) mit krummlinigen eingekehlten Seiten (24, 25) aufweist, die drei bevorzugte separate und voneinander beabstandete Kontaktbereiche darstellen, nämlich einen ersten Kontaktbereich (18) an der inneren Kortikalen (19) des Knochens, einen zweiten Kontaktbereich (20) an der postero-externen Kortikalen (21) des Knochens und einen dritten Kontaktbereich (22) an der antero-externen Kortikalen (23) des Knochens, wobei der zweite (20) und der dritte (22) Kontaktbereich mit dem ersten Kontaktbereich (18) durch eingekehlte konkave Seiten (24, 25) verbunden sind, die dazu vorgesehen sind, in einem Abstand vom Knochen zu verbleiben, wobei die drei Kontaktbereiche (18, 20, 22) in die innere anatomische Kontur des kortikalen Knochens (9) eingreifen.

7. Prothese nach Anspruch 6, **dadurch gekennzeichnet, daß** der im Knochen liegende Bereich der Prothese einen distalen Teil (17) mit einer im wesentlichen drehkonischen Form mit gerader Achse aufweist.

8. Prothese nach Anspruch 7, **dadurch gekennzeichnet, daß** der distale Teil (17) eine glatte und polierte äußere Oberfläche aufweist.

9. Prothese nach einem der Ansprüche 6 bis 8, **dadurch gekennzeichnet, daß** eine Zentrierung (26) aus einem elastischen, deformierbaren Material am Ende des distalen Teils (17) des Implantats oder des zugeordneten Einsetzwerkzeuges vorgesehen ist, zum Zentrieren des distalen Teils (17) in dem Markkanal (8) des Oberschenkelknochens (9), ohne die durch die Kontaktbereiche (18, 20, 22) des proximalen Teils (16) der Prothese hergestellten Kontakte zu beeinträchtigen.

## Claims

1. Hip prosthesis adapted to be fitted into the upper end of a femur (9), comprising a prosthesis head (1) joined by a neck (2) to a prosthesis stem (3) to be accommodated and cemented into a longitudinal passage (8) formed in the femur (9), the head (1) and the neck (2) constituting an extra-bone area intended to remain outside the bone (9) and joined by a transition area (4) to the stem (3) which constitutes an intra-bone area, **characterised in that**, in the vicinity of the transition area (4), the extra-bone area comprises an annular surface (5) made from a material having an affinity with bone or with the new fibrous membranes that form naturally around foreign bodies in the human body, so that, after the prosthesis is fitted, a fibrous seal (14) forms naturally between the upper end (15) of the femur (9) and said annular surface (5), the fibrous seal (14) formed in this way opposing penetration of foreign bodies and wear products between the bone (9) and the prosthesis stem (3).

2. Prosthesis according to claim 1, **characterised in that** the extra-bone area annular surface (5) is extended, beyond the transition area (4), by an intra-bone area annular surface (6) made from a material having an affinity with bone or with the new fibrous membranes that form naturally around foreign bodies, enabling re-implantation and packing of spongy bone between the prosthesis and cortical bone of the femur, constituting a bone seal (13) complementing and continuous with said fibrous seal (14) ahead of the cemented fixing area of the prosthesis stem (3).

3. Prosthesis according to claim 2, **characterised in that** the intra-bone area annular surface (6) is in the form of an annular groove to favor insertion of spongy bone when the prosthesis is implanted.

4. Prosthesis according to any one of claims 1 to 3, **characterised in that** the annular surface (5, 6) is made from a rehabitable porous or rough material, optionally partly covered with a phosphate-calcium substance.

5. Prosthesis according to any one of claims 1 to 4, **characterised in that** a removable protective cap (7) covers the extra-bone area (1, 2) of the prosthesis as far as the fixing area of the stem (3) to be cemented, to protect the prosthesis peripheral annular surface (5, 6) that subsequently constitutes the seal from said cement (10), the cap (7) being removed after said prosthesis is cemented in place.

6. Prosthesis according to any one of claims 1 to 5, **characterised in that** the prosthesis intra-bone area has an anatomically shaped metaphyseal cortex proximal part (16) with a curvilinear triangular cross-section (16a, 16b) with hollowed out flanks (24, 25), defining three separate and spaced apart preferential contact areas, namely a first contact area (18) on the internal cortex (19) of the bone, a second contact area (20) on the posterior external cortex (21) of the bone, and a third contact area (22) on the anterior external cortex (23) of the bone, said second (20) and third (22) contact areas being joined to said first contact area (18) by hollowed out concave flanks (24, 25) adapted not to come into contact with the bone, the three contact areas (18, 20, 22) being circumscribed within the internal anatomical contour of the cortical bone (9).

7. Prosthesis according to claim 6, **characterised in that** said prosthesis intra-bone area comprises a distal part (17) the general shape of which is a cone having a rectilinear axis.

8. Prosthesis according to claim 7, **characterised in that** the distal part (17) has a smooth and polished peripheral surface.

9. Prosthesis according to any one of claims 6 to 8, **characterised in that** an elastically deformable material centering member (26) is fitted to the end of said implant distal part (17) or a corresponding ancillary member to center said distal part (17) in the medullary canal (8) of the femur (9) without disturbing contact at said contact areas (18, 20, 22) of the prosthesis proximal part (16).
